# EUROPEAN PATENT APPLICATION

(11) **EP 4 740 906 A1**
(43) Date of publication of application: **13.05.2026**
(21) Application number: 24836371.5
(22) Date of filing: 05.07.2024
(51) Int. Cl.: A61C 19/04, A61C 9/00, A61B 5/00, A61B 6/51, A61B 6/03, A61B 6/46, G06F 17/00, G06F 30/20

(54) **SCAN IMAGE PROCESSING METHOD OF INTRAORAL SCANNER, DEVICE THEREFOR, AND RECORDING MEDIUM ON WHICH INSTRUCTIONS THEREOF ARE RECORDED**

(30) Priority: 06.07.2023 KR 20230087607; 04.07.2024 KR 20240088170
(71) Applicant: Medit Corp., Seoul 07207 (KR)
(72) Inventor: KO, Seunghee, Seoul 07207 (KR)
(74) Representative: MFG Patentanwälte Meyer-Wildhagen Meggle-Freund Gerhard PartG mbB
(86) International application number: PCT/KR2024/009552
(87) International publication number: WO 2025/009924

(57) **Abstract**

A method of the present disclosure includes: arranging a three-dimensional (3D) image of a target oral cavity in a 3D space; receiving an input for selecting a plurality of teeth of the target oral cavity; arranging first library teeth corresponding to the 3D image of the target oral cavity in the 3D space; moving a plurality of second library teeth in the 3D space such that the plurality of second library teeth corresponding to the plurality of teeth among the first library teeth are arranged in a space corresponding to the plurality of teeth among the 3D image of the target oral cavity; and displaying the 3D image of the target oral cavity and the plurality of second library teeth to overlap each other.

## Description

### TECHNICAL FIELD

The present disclosure relates to a scan image processing method of an intraoral scanner, a device therefor, and a recording medium recording instructions thereof.

### BACKGROUND

A three-dimensional (3D) image of an oral cavity refers to scan data obtained via a 3D scanner that scans teeth and an oral cavity, or an object obtained via modeling or reconstructing the same. This may be used for obtaining a 3D image of the oral cavity of a patient so as to design a dental prosthesis or implant or to manufacture an orthodontic appliance during a prosthetic treatment such as an inlay, onlay, crown, implant or the like as well as other dental treatments such as an orthodontic treatment or the like.

Conventionally, a dental prosthesis, implant, or orthodontic appliance has been fabricated by hand after manually modeling an oral cavity using alginate or the like. Recently, a digital method that obtains 3D intraoral scan data of a patient using a 3D scanner, designs a dental prosthesis, implant, or orthodontic appliance using a computer, and 3D print the same has been widely used.

### DISCLOSURE

### TECHNICAL PROBLEM

Various embodiments of the present disclosure provide a method of displaying a plurality of first library teeth selected by a user to overlap a three-dimensional (3D) image of an oral cavity.

Various embodiments of the present disclosure provide a method of determining position information of a plurality of library teeth using segmentation and/or margin lines that separate a tooth region and a gingival region in a 3D image of an oral cavity, and arranging the library teeth.

Various embodiments of the present disclosure provide a method of displaying a plurality of library teeth in a 3D image of an oral cavity so that a user may edit the plurality of library teeth without switching a screen.

Various embodiments of the present disclosure provide a method of forming a customized library tooth using actual tooth data of a patient (3D image of oral cavity).

The problems to be solved by the present disclosure are not limited to the above-described problems, and other problems that are not mentioned above will be clearly understood by those skilled in the art from the following description.

### TECHNICAL SOLUTION

In one embodiment, an image processing method performed by an electronic device may include arranging a three-dimensional (3D) image of a target oral cavity in a 3D space; receiving an input for selecting a plurality of teeth of the target oral cavity; arranging first library teeth corresponding to the 3D image of the target oral cavity in the 3D space; moving a plurality of second library teeth in the 3D space such that the plurality of second library teeth corresponding to the plurality of teeth among the first library teeth are arranged in a space corresponding to the plurality of teeth among the 3D image of the target oral cavity; and displaying the 3D image of the target oral cavity and the plurality of second library teeth to overlap each other.

In one embodiment, the method further includes obtaining a plurality of margin lines corresponding to the plurality of teeth, respectively, wherein the arranging the first library teeth in the 3D space includes, based on the plurality of margin lines, arranging the first library teeth in the 3D space, and wherein each of the plurality of margin lines is a boundary at which an intact tooth begins to emerge from a gingival region of the 3D image of the target oral cavity.

In one embodiment, wherein the obtaining the plurality of margin lines includes, based on a boundary between a tooth region and a gingival region obtained as a result of segmentation, obtaining the plurality of margin lines; or based on a user input, obtaining the plurality of margin lines.

In one embodiment, the arranging the first library teeth in the 3D space includes: identifying a tooth number corresponding to a tooth region obtained as a result of segmentation; and based on the tooth numbers, arranging the first library teeth in the 3D space.

In one embodiment, the method further includes generating the first library teeth.

In one embodiment, the generating the first library teeth includes: based on mesh information of at least a portion of the first library teeth corresponding to a tooth region among the tooth region and a gingival region included in the 3D image of the target oral cavity, enlarging the first library teeth in a direction of the gingival region.

In one embodiment, the generating the first library teeth further includes, based on the plurality of margin lines, processing the enlarged region in the enlarged first library teeth.

In one embodiment, the receiving the input for selecting the plurality of teeth of the target oral cavity includes receiving an input for selecting a tooth number for each of the plurality of teeth of the target oral cavity.

In one embodiment, the moving the plurality of second library teeth in the 3D space includes: based on each of the plurality of margin lines, generating first position information including a center point of a first bounding box for each tooth of the plurality of second library teeth; generating second position information including a center point of a second bounding box for each tooth of the 3D image of the target oral cavity; and based on an origin positioned in a dental arch of the target oral cavity, generating a correlation between the first position information and the second position information for each of the plurality of second library teeth.

In one embodiment, the generating the correlation includes, based on the origin, generating a tooth-specific transform matrix indicating a correlation between the first position information of each of the plurality of second library teeth and the second position information having the same tooth numbers as each of the plurality of the second library teeth.

In one embodiment, the displaying the 3D image of the target oral cavity and the plurality of second library teeth to overlap each other includes, based on the tooth-specific transform matrix, displaying the plurality of second library teeth to overlap the 3D image of the target oral cavity having the same tooth numbers as the plurality of second library teeth, respectively, in the 3D space.

In one embodiment, the origin is a point between two central incisors in the dental arch.

In one embodiment, the displaying the 3D image of the target oral cavity and the plurality of second library teeth to overlap each other includes, based on a tooth-specific transform matrix corresponding to a removed tooth, displaying a library tooth corresponding to the removed tooth to overlap the 3D image of the target oral cavity.

In one embodiment, the tooth-specific transform matrix corresponding to the removed tooth is generated based on second position information including a point in a dental arch that corresponds to the tooth number, and the library tooth corresponding to the removed tooth includes a dental prosthesis corresponding to the removed tooth.

In one embodiment, the displaying the 3D image of the target oral cavity and the plurality of second library teeth to overlap each other includes, based on a tooth-specific transform matrix corresponding to a prep tooth, displaying a library tooth corresponding to the prep tooth to overlap the 3D image of the target oral cavity.

In one embodiment, the tooth-specific transform matrix corresponding to the prep tooth is generated based on second position information of which a center point of a second bounding box is changed based on a difference between a height of a highest point of the prep tooth and a height of a highest point of the library tooth corresponding to the prep tooth, and the library tooth corresponding to the prep tooth includes a dental prosthesis corresponding to the prep tooth.

In one embodiment, the displaying the 3D image of the target oral cavity and the plurality of second library teeth to overlap each other includes, based on a determination that the plurality of second library teeth corresponding to the plurality of teeth is connected via a connector, displaying the connector between the plurality of second library teeth.

In another embodiment, an electronic device includes: a processor; a network interface communicatively connected to an intraoral scanner; a display; a memory; and a computer program loaded into the memory and executed by the processor, wherein the computer program is configured to: arrange a three-dimensional (3D) image of a target oral cavity in a 3D space; receive an input for selecting a plurality of teeth of the target oral cavity; arrange first library teeth corresponding to the 3D image of the target oral cavity in the 3D space; move a plurality of second library teeth in the 3D space such that the plurality of second library teeth corresponding to the plurality of teeth among the first library teeth are arranged in a space corresponding to the plurality of teeth among the 3D image of the target oral cavity; and display the 3D image of the target oral cavity and the plurality of second library teeth to overlap each other.

In another embodiment, a non-transitory computer-readable recording medium recording a computer program to be executed by a processor, includes: an instruction for arranging a three-dimensional (3D) image of a target oral cavity in a 3D space; an instruction for receiving an input for selecting a plurality of teeth of the target oral cavity; an instruction for arranging first library teeth corresponding to the 3D image of the target oral cavity in the 3D space; an instruction for moving a plurality of second library teeth in the 3D space such that the plurality of second library teeth corresponding to the plurality of teeth among the first library teeth are arranged in a space corresponding to the plurality of teeth of the 3D image of the target oral cavity; and an instruction for displaying the 3D image of the target oral cavity and the plurality of second library teeth to overlap each other.

### ADVANTAGEOUS EFFECTS

According to the present disclosure in some embodiments, a method, which displays a plurality of first library teeth selected by a user so as to overlap a three-dimensional (3D) image of an oral cavity, is provided.

According to the present disclosure in some embodiments, a method, which determines position information of a plurality of library teeth using segmentation and/or margin lines that separate a tooth region and a gingival region in a 3D image of an oral cavity, and arranges the library teeth, is provided.

According to the present disclosure in some embodiments, a method, which displays a plurality of library teeth so as to overlap a 3D image of an oral cavity so that a user may edit the plurality of library teeth without switching a screen, is provided.

According to the present disclosure in some embodiments, a method, which forms a customized library tooth using actual tooth data of a patient (3D image of oral cavity), is provided.

### DESCRIPTION OF DRAWINGS

FIG. 1 is a diagram illustrating a scanning environment according to embodiments of the present disclosure.
FIG. 2 is a block diagram illustrating an electronic device and an intraoral scanner shown in FIG. 1
FIG. 3 is a diagram illustrating the intraoral scanner described with reference to FIG. 2.
FIGS. 4A and 4B are diagrams illustrating three-dimensional (3D) images of a target oral cavity according to the present disclosure.
FIG. 4C is a diagram illustrating first library teeth according to the present disclosure.
FIG. 5 is a diagram illustrating a margin line according to the present disclosure.
FIG. 6 is a diagram illustrating a method of selecting a plurality of teeth according to the present disclosure.
FIG. 7 is a diagram illustrating second position information in a 3D image of an oral cavity according to the present disclosure.
FIG. 8 is a diagram illustrating first position information of a library tooth according to the present disclosure.
FIG. 9 is a diagram illustrating a method of overlapping the library tooth described with reference to FIG. 8 to the 3D image of the oral cavity described with reference to FIG. 7.
FIG. 10 is a flowchart illustrating a method of displaying a library tooth so as to overlap a 3D image of an oral cavity, by a server according to the present disclosure.

### MODE FOR INVENTION

Embodiments of the present disclosure are illustrated for describing the technical spirit of the present disclosure. The scope of rights according to the present disclosure is not limited to the embodiments described below or to the detailed descriptions of these embodiments.

All technical or scientific terms used herein have meanings that are generally understood by a person having ordinary knowledge in the art to which the present disclosure pertains, unless otherwise specified. The terms used herein are selected for more clear illustration of the present disclosure, and are not intended to limit the scope of rights in accordance with the present disclosure.

The expressions "include," "provided with," "have" and the like used herein should be understood as open-ended terms connoting the possibility of inclusion of other embodiments, unless otherwise mentioned in a phrase or sentence including the expressions.

A singular expression can include meanings of plurality, unless otherwise mentioned, and the same is applied to a singular expression recited in the claims. The terms "first," "second," etc. used herein are used to identify a plurality of components from one another, and are not intended to limit the order or importance of the relevant components.

The term "unit" used in the present disclosure means a software component or hardware component, such as a field-programmable gate array (FPGA) and an application specific integrated circuit (ASIC). However, a "unit" is not limited to software and hardware and it may be configured to be in an addressable storage medium or may be configured to run on one or more processors. For example, a "unit" may include components, such as software components, object-oriented software components, class components, and task components, as well as processors, functions, attributes, procedures, subroutines, segments of program codes, drivers, firmware, micro-codes, circuits, data, databases, data structures, tables, arrays, and variables. Functions provided in components and "unit" may be combined into a smaller number of components and "units" or further subdivided into additional components and "units."

The expression "based on" used herein is used to describe one or more factors that influences a decision, an action of judgment or an operation described in a phrase or sentence including the relevant expression, and this expression does not exclude an additional factor influencing the decision, the action of judgment or the operation.

When a certain component is described as "coupled to" or "connected to" another component, this should be understood as meaning that the certain component may be coupled or connected directly to the other component or that the certain component may be coupled or connected to the other component via a new intervening component.

In the following description, embodiments of the present disclosure will be described with reference to the accompanying drawings. In the accompanying drawings, like or relevant components are indicated by like reference numerals. In the following description of embodiments, repeated descriptions of the identical or relevant components will be omitted. However, even if a description of a component is omitted, such a component is not intended to be excluded in an embodiment.

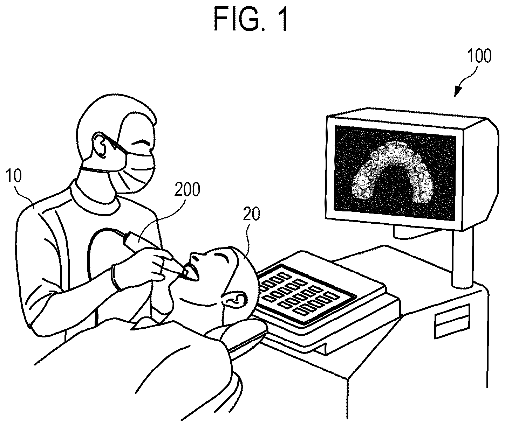

FIG. 1 is a diagram illustrating a scanning environment according to embodiments of the present disclosure. Specifically, the scanning environment illustrated in FIG. 1 is an environment where an image of an oral cavity of a patient is obtained using an intraoral scanner 200 according to embodiments of the present disclosure and, herein, the intraoral scanner 200 may be a dental device for obtaining an image of an oral cavity of a target object 20 (e.g., patient).

As illustrated in FIG. 1, a user 10 (e.g., dentist, dental hygienist) may obtain an image of the oral cavity of the target object 20 from the target object 20 by using the intraoral scanner 200. As another example, the user 10 may obtain an image of the oral cavity of the target object 20 from a diagnostic model (e.g., plaster model, impression model) replicating the oral cavity of the target object 20. In the following description, for ease of description, description will be provided based on an example of obtaining an image of the oral cavity of the target object 20, but the scope of the present disclosure is not limited to the example. In addition, a region to be captured to obtain an image is not limited to the oral cavity of the target object 20, and images of other regions of the target object 20 (e.g., ears of target object 20) may also be acquired.

In the following description, operation of the components illustrated in FIG. 1 will be described in detail.

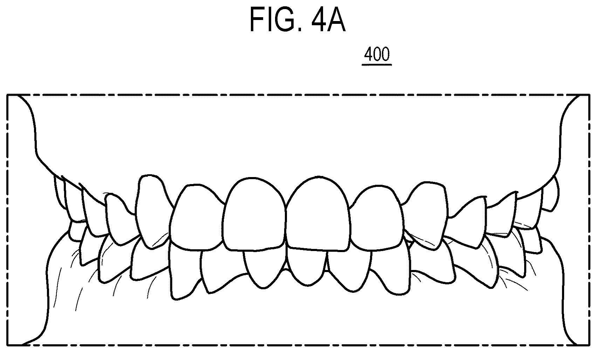

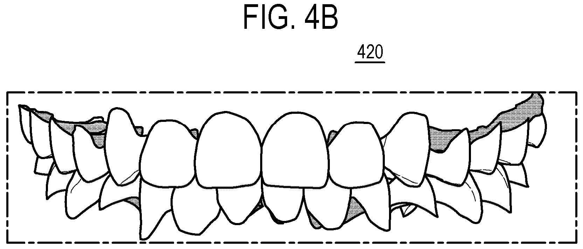

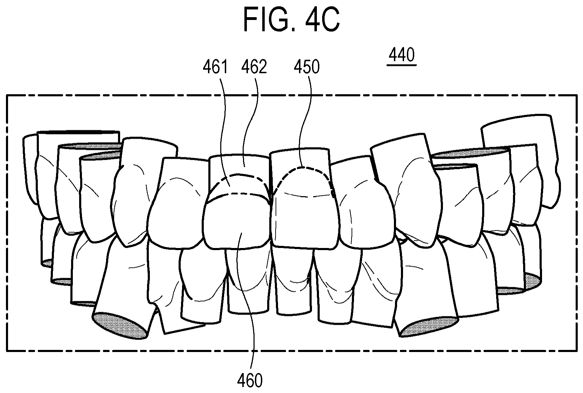

An electronic device 100 illustrated in FIG. 1 may receive two-dimensional (2D) images of the oral cavity of the target object 20 from the intraoral scanner 200. Here, the electronic device 100 may communicate with the intraoral scanner 200 via a wired or wireless communication network. In addition, the electronic device 100 may generate a 3D image of the oral cavity by modeling an internal structure of the oral cavity based on the received 2D images of the oral cavity, and a detailed operation related thereto will be described with reference to FIG. 4 later on. The 3D image of the oral cavity generated in this manner may be displayed to the user 10 or the target object 20 via a display of the electronic device 100. In this instance, the user 10 may give appropriate medical services to the target object20 by referring to the 3D image displayed on the display of the electronic device 100.

In some embodiments, the electronic device 100 may receive a 3D image of an oral cavity generated by the intraoral scanner 200. Here, the intraoral scanner 200 may scan the oral cavity of the target object 20 and obtain 2D images of the oral cavity, and may generate a 3D image of the oral cavity based on the obtained 2D images of the oral cavity. That is, irrespective of where an operation of generating a 3D image is processed, it should be noted that the operation is included in the scope of the present disclosure.

In addition, the electronic device 100 may be communicatively connected to a cloud server (not illustrated). In this case, the electronic device 100 may transmit the 2D images or 3D image of the oral cavity of the target object 20 to the cloud server, and the cloud server may store the 2D images or the 3D image of the oral cavity of the target object 20 received from the electronic device 100.

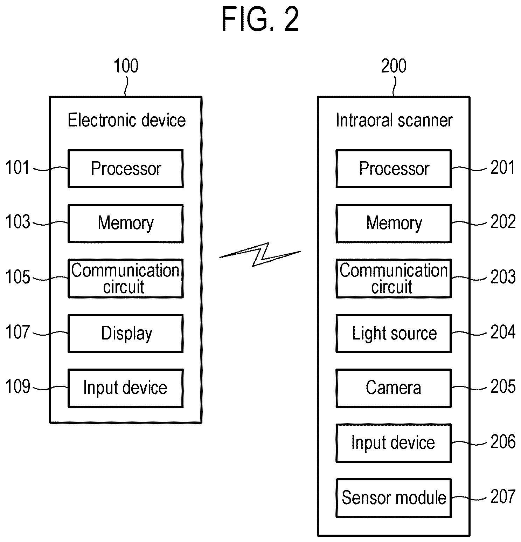

The electronic device 100 that has been described may be implemented as a computing device, and an example of the computing device will be described later in detail with reference to FIG. 2.

The intraoral scanner 200 illustrated in FIG. 1 may have a form capable of being inserted into and removed from an oral cavity, and may be a handheld scanner of which a scan distance and a scan angle may be freely adjusted by the user 10.

The intraoral scanner 200 may be inserted into an oral cavity and perform contactless scanning of the internal oral cavity so as to obtain an image of the oral cavity. The image of the oral cavity may include at least one tooth, the gingiva, or an artificial structure capable of being inserted into the oral cavity (e.g., orthodontic appliance including a bracket and wire, an implant, a denture, an orthodontic accessory configured to be inserted into an oral cavity, etc.). Specifically, the intraoral scanner 200 may project light onto the oral cavity of the target object 20 by using a light source (or projector), and may receive light reflected from the oral cavity of the target object 20 via a camera (or at least one image sensor).

In addition, the intraoral scanner 200 may obtain a surface image of the oral cavity of the target object 20 as a 2D images based on information received via the camera. Here, the surface image of the oral cavity of the target object 20 may include at least one of the teeth, gingiva, artificial structure, cheek, tongue, or lip of the target object 20.

As described above, in some embodiments, the intraoral scanner 200 may scan an oral cavity to obtain a 2D images of the oral cavity, and may generate a 3D image of the oral cavity based on the obtained 2D images of the oral cavity.

The intraoral scanner 200 that has been described may be implemented as a computing device, and an example of the computing device will be described in detail later with reference to FIG. 2.

FIG. 2 is a block diagram illustrating the electronic device 100 and the intraoral scanner 200 shown in FIG. 1. The block diagram illustrated in FIG. 2 merely shows an exemplary embodiment for achieving an objective of the present disclosure, and some components may be added or deleted as occasion demands. In addition, the components of the block diagram illustrated in FIG. 2 are functional elements divided based on their functions, and it should be noted that a plurality of different components may be integrated and implemented in an actual physical environment. In the following description, each component of the block diagram will be described in detail.

The electronic device 100 illustrated in FIG. 2 may include at least one processor 101, at least one memory 103, a communication circuit 105, a display 107, and/or an input device 109. As described above, at least one of the components included in the electronic device 100 may be omitted or other components may be added to the electronic device 100. Additionally or alternatively, some components may be integrated and implemented, or may be implemented as a single entity or a plurality of entities. At least some of the components in the electronic device 100 may be connected to each other via a bus, a general purpose input/output (GPIO), a serial peripheral interface (SPI), or a mobile industry processor interface (MIPI), or the like, and may transmit and receive data and/or signals.

One or more processors 101 of the electronic device 100 may be configured to perform operations or data processing in association with control of the components of the electronic device 100 (e.g., memory 103) and/or communication. The one or more processors 101, for example, may be operatively connected to the components of the electronic device 100. In addition, the one or more processors 101 may load instructions or data received from other components of the electronic device 100 into the one or more memories 103, may process the instructions or data stored in the one or memories 103, and may store the result data.

Subsequently, the one or more memories 103 of the electronic device 100 may store various data, instructions, and/or information. Specifically, the one or more memories 103 may store instructions associated with operations of the processor 101 as a computer program. In addition, the one or more memories 103 may store correlation models built according to a machine learning algorithm. In addition, the one or more memories 103 may store data (e.g., 2D image or 3D image of oral cavity) received from the intraoral scanner 200.

Subsequently, a communication circuit (network interface) 105 of the electronic device 100 may establish a wired or wireless communication channel with an external device (e.g., intraoral scanner 200, cloud server (not illustrated)), and may transmit and receive various data with the external device. In some embodiments, to communicate with an external device in a wired manner, the communication circuit 105 may include at least one port to connect to the external device via a wired cable. In this case, the communication circuit 105 may communicate with the external device connected via at least one port in a wired manner. In some embodiments, the communication circuit 105 may include a cellular communication module and may be configured to be connected to a cellular network (e.g., 3G, LTE, 5G, Wibro, or Wimax). In some embodiments, the communication circuit 105 may include a short-range communication module and may perform data transmission and reception with an external device via short-range communication (e.g., Wi-Fi, Bluetooth, Bluetooth low energy (BLE), UWB). In some embodiments, the communication circuit 105 may include a contactless communication module for contactless communication. Here, the contactless communication may include at least one contactless short range communication technology, for example, near field communication (NFC), radio frequency identification (RFID) communication, or magnetic secure transmission (MST) communication. In addition to the above-described examples, the electronic device 100 may be implemented in various publicly known methods for communication with an external device, and it should be noted that the scope of the present disclosure is not limited to the above-described examples.

Subsequently, the display 107 of the electronic device 100 may display various screens under the control of the processor 101. Here, under the control of the processor 101, a 2D images of the oral cavity of the target object 20 received from the intraoral scanner 200 and/or a 3D image obtained by modeling an internal structure of the oral cavity in three dimension may be displayed on the display 107. In this instance, to display the 2D image and/or 3D image of the oral cavity on the display 107, for example, a web browser or a dedicated application may be installed in the electronic device 100. In some embodiments, the web browser or dedicated application may be implemented so as to provide a function of editing, storing, and deleting the 2D image and/or 3D image of the oral cavity to the user 10 via a user interface.

Subsequently, the input device 109 of the electronic device 100 may receive instructions or data to be used for a component (e.g., processor 101) of the electronic device 100 from outside the electronic device 100 (e.g., user). The input device 109 may include, for example, a microphone, a mouse, or a keyboard. In some embodiments, the input device 109 may be coupled with the display 107 and may be implemented in the form of a touch sensor panel capable of recognizing contact or approach of various external objects. However, the scope of the present disclosure is not limited to the above-described examples, and various publicly known input devices 109 may be included in the scope of the present disclosure for user convenience.

The intraoral scanner 200 illustrated in FIG. 2 may include a processor 201, a memory 202, a communication circuit 203, a light source 204, a camera 205, and/or an input device 206. As described above, at least one of the components included in the intraoral scanner 200 may be omitted or other components may be added to the intraoral scanner 200. Additionally or alternatively, some components may be integrated and implemented, or may be implemented as a single entity or a plurality of entities. At least some of the components in the intraoral scanner 200 may be connected to each other via a bus, a general purpose input/output (GPIO), a serial peripheral interface (SPI), or a mobile industry processor interface (MIPI), or the like, and may transmit and receive data and/or signals.

The processor 201 of the intraoral scanner 200 may be a component capable of performing operations or data processing in association with control of the components of the intraoral scanner 200 and/or communication, and may be operatively connected to the components of the intraoral scanner 200. In addition, the processor 201 may load instructions or data received from other components of the intraoral scanner 200 into the memory 202, may process the instructions or data stored in the memory 202, and may store the result data.

Subsequently, the memory 202 of the intraoral scanner 200 may store instructions associated with the above-described operations of the processor 201.

Subsequently, the communication circuit 203 of the intraoral scanner 200 may establish a wired or wireless communication channel with an external device (e.g., electronic device 100), and may transmit and receive various data with the external device. In some embodiments, to communicate with the external device in a wired manner, the communication circuit 203 may include at least one port to connect to the external device via a wired cable. In this case, the communication circuit 203 may communicate with the external device connected via at least one port in a wired manner. In some embodiments, the communication circuit 203 may include a cellular communication module and may be configured to be connected to a cellular network (e.g., 3G, LTE, 5G, Wibro, or Wimax). In some embodiments, the communication circuit 203 may include a short-range communication module and may perform data transmission and reception with an external device via short-range communication (e.g., Wi-Fi, Bluetooth, Bluetooth low energy (BLE), UWB). In some embodiments, the communication circuit 203 may include a contactless communication module for contactless communication. Here, the contactless communication may include at least one contactless short range communication technology, for example, near field communication (NFC), radio frequency identification (RFID) communication, or magnetic secure transmission (MST) communication. In addition to the above-described examples, the intraoral scanner 200 may be implemented in various publicly known methods for communication with an external device, and it should be noted that the scope of the present disclosure is not limited to the above-described examples.

Subsequently, the light source 204 of the intraoral scanner 200 may project light toward the oral cavity of the target object 20. For example, light projected from the light source 204 may be structured light with a predetermined pattern (e.g., a stripe patten including a pattern of a series of straight lines with different colors). Here, the pattern of the structured light, for example, may be generated using a pattern mask or digital micro-mirror device (DMD), but the present disclosure is not limited thereto.

Subsequently, the camera 205 of the intraoral scanner 200 may receive reflected light that is reflected by the oral cavity of the target object 20, and may obtain a 3D image of the oral cavity of the target object 20. Here, the camera 205 may include a left camera corresponding to a left-eye view and a right camera corresponding to a right-eye view in order to build a 3D image according to an optical triangulation method. In addition, the camera 205 may include at least one image sensor such as a CCD sensor or a CMOS sensor.

Subsequently, the input device 206 of the intraoral scanner 200 may receive a user input for controlling the intraoral scanner 200. For example, the input device 206 may include a button for receiving a push operation by the user 10, a touch panel for detecting a touch by the user 10, and a voice recognition device including a microphone. In this instance, the user 10 may use the input device 206 to control starting or stopping of scanning.

Operation of the intraoral scanner 200 controlled by the input device 206 will be described in detail. The intraoral scanner 200 may receive a user input for starting scanning via the input device 206 of the intraoral scanner 200 or the input device 206 of the electronic device 100, and may start scanning according to processing performed by the processor 201 of the intraoral scanner 200 or the processor 201 of the electronic device 100. Here, when the user 10 uses the intraoral scanner 200 to scan the internal oral cavity of the target object 20, the intraoral scanner 200 may generate a 2D image of the oral cavity of the target object 20 and may transmit the 2D image of the oral cavity of the target object 20 to the electronic device 100 in real time. In this instance, the electronic device 100 may display the received 2D image of the oral cavity of the target object 20 on the display 107. In addition, based on the 2D images of the oral cavity of the target object 20, the electronic device 100 may generate (construct) a 3D image of the oral cavity of the target object 20, and may display the 3D image of the oral cavity on the display 107. In this instance, the electronic device 100 may display the 3D image that is being generated on the display 107 in real time.

Subsequently, a sensor module 207 of the intraoral scanner 200 may detect an operation state of the intraoral scanner 200 or an external environment state (e.g., user's motion), and may generate an electric signal corresponding to the detected state. The sensor module 207, for example, may include at least one of a gyro sensor, an acceleration sensor, a gesture sensor, a proximity sensor, or an infrared ray sensor. In this instance, the user 10 may use the sensor module 207 to control starting or stopping of scanning. Specifically, for example, in the case in which the user 10 holds the intraoral scanner 200 with a hand and moves the same, the processor 201 may control the intraoral scanner 200 to start a scanning operation when an angular speed measured via the sensor module 207 exceeds a predetermined value.

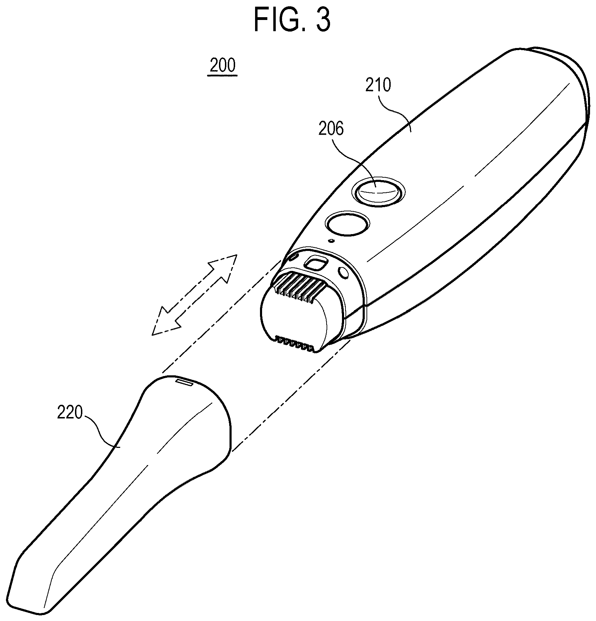

In the following description, the above-described intraoral scanner 200 will be described in detail with reference to FIG. 3. FIG. 3 is a diagram illustrating the intraoral scanner 200 described with reference to FIG. 2.

The intraoral scanner 200 illustrated in FIG. 3 may include a main body 210 and a probe tip 220. Here, the main body 210 of the intraoral scanner 200 may be formed in a shape that allows the user 10 to easily grip the same for use, and the probe tip 220 may be formed in a shape that allows easy insertion into and removal from the oral cavity of the target object 20. In addition, the main body 210 may be coupled with and separated from the probe tip 220. In addition, inside the main body 210, the components of the intraoral scanner 200 that has been described with reference to FIG. 2 may be disposed. An opening part that is opened so that light output from the light source 204 is projected onto the target object 20 may be formed in one end of the main body 210. The light projected via the opening end may be reflected by the target object 20 and enter the opening part again. Here, the reflected light that enters the opening part may be captured by the camera so that an image of the target object 20 may be generated. In addition, the user 10 may start scanning by using the input device 206 (e.g., button) of the intraoral scanner 200. For example, when the user 10 touches or presses the input device 206, light from the light source 204 may be projected onto the target object 20.

With reference to FIGS. 1 to 3, the scanning environments according to some embodiments of the present disclosure and the components included therein have been described in detail.

In the present disclosure, a library tooth is a sample tooth (standard tooth) used for manufacturing a dental prosthesis, implant, orthodontic appliance or the like and may have a typical tooth shape. The library tooth may be represented as a 3D image, and may be displayed based on mesh information. The library tooth may have one sample tooth (standard tooth) for each tooth number. Each sample tooth may include general characteristics of a natural tooth shape corresponding to its tooth number. For example, when a predetermined tooth of a patient is lost or is removed for treatment and needs a dental prosthesis for the lost or removed tooth, the shape of a corresponding sample tooth may be used to efficiently make a dental prosthesis having a natural shape. In addition, a 3D image of an oral cavity may be captured by the intraoral scanner 200 and a mesh may have a relatively low degree of completion. When the mesh has a low degree of completion, it may be improper to manufacture a dental prosthesis, implant, orthodontic appliance, or the like via 3D printing. On the other hand, the library tooth may be a dental model having a mesh with a high degree of completion. Therefore, manufacturing a dental prosthesis, implant, orthodontic application or the like by modifying a library tooth may be proper to use a 3D printing scheme. By arranging a library tooth in a 3D image of a patient's oral cavity, the library tooth may become an intermediate model appropriate for manufacturing a dental prosthesis, implant, orthodontic appliance or the like in a digital manner.

In another embodiment, a library tooth may be generated based on a 3D image of an oral cavity. The library tooth may be generated based on mesh information corresponding to a tooth region among the tooth region and a gingival region that are separated by segmentation in the 3D image of the oral cavity. Through the above, the electronic device 100 may generate a library tooth similar to an actual tooth of a patient.

In another embodiment, a library tooth may be generated based on a past 3D image of the oral cavity of a predetermined target. For example, a 3D image of the oral cavity of the patient, which was obtained before a predetermined tooth of the patient was lost or removed, may be stored in the memory 103. The electronic device 100 may generate a library tooth based on the 3D image of the oral cavity including the intact predetermined tooth. In this instance, the library tooth may include the natural tooth shape of the patient as it is.

The library tooth may be utilized for manufacturing a dental prosthesis, implant, orthodontic appliance, dental appliance or the like and thus, the time and efforts needed for manufacturing the dental prosthesis, implant, orthodontic appliance, dental appliance or the like may be reduced, the accuracy and productivity of the dental prosthesis, implant, orthodontic appliance, dental appliance or the like may be increased, and the amount of computing resources consumed to obtain the equivalent result may be reduced.

In the present disclosure, a library tooth previously designed in some degree for each tooth number (dental formula) may be used. To manufacture a dental prosthesis, implant, and orthodontic appliance, the library tooth may be displayed so as to overlap (be aligned with) a 3D image of an oral cavity. When a process of overlapping or arranging the library tooth in the 3D image is performed manually, the fatigue of a dentist or a dental technician may be increased and the accuracy and productivity of an output may be reduced. Accordingly, the amount of computing resources consumed to obtain an equivalent result may be increased.

In addition, when the number of library teeth to be used is more than 2, the library teeth may be selected one by one, and may need to be edited in a separate editing screen and arranged manually. Therefore, in the following description, a method of arranging two or more library teeth in a 3D image of an oral cavity and displaying the same, and editing the two or more library teeth in the same screen without switching a screen will be described in detail.

In an embodiment, the electronic device 100 may arrange a 3D image of a target oral cavity in a 3D space.

FIGS. 4A and 4B are diagrams illustrating a three-dimensional (3D) image of a target oral cavity of the present disclosure.

FIG. 4A illustrates an example of a 3D image 400 of an oral cavity. The 3D image 400 of the oral cavity may include a gingival region and a tooth region. The electronic device 100 may separate the gingival region and the tooth region in the 3D image 400 via segmentation. The segmentation may be image segmentation. The image segmentation is a technical process that divides a digital image into individually distinguishable parts or objects. The process aims to divide the image into a plurality of meaningful regions so that each part is easily analyzed and processed. Specifically, the image segmentation is performed by classifying pixels into groups, each group sharing a same attribute or class. The regions obtained via division, for example, may include an object boundary, a surface, or other important factors.

FIG. 4B illustrates an example of a tooth region 420 only obtained from the 3D image 400 of an oral cavity. The electronic device 100 may generate a library tooth using the tooth region 420. By generating a library tooth from a 3D image of an oral cavity of a predetermined target object obtained by scanning the oral cavity, the electronic device 100 may generate a library tooth appropriate for individual characteristics of the target object. According to an embodiment, the electronic device 100 may generate the surface of the library tooth using the tooth region 420. The surface of the library tooth may be composed of a mesh. According to an embodiment, the inside of the library tooth may be represented as a hollow space. According to another embodiment, the inside of the library tooth may be represented as a filled form based on mesh information related to the tooth.

FIG. 4C is a diagram illustrating first library teeth of the present disclosure.

A library tooth may be provided in a sample tooth form having a typical tooth shape and/or may be provided in a tooth form generated based on a 3D image of the oral cavity of a predetermined target object. Therefore, the electronic device 100 may obtain, from the memory 103, a library tooth provided in a sample tooth form, or may obtain a library tooth newly generated based on the 3D image of the predetermined target oral cavity.

FIG. 4C illustrates first library teeth 440 generated based on the tooth region 420 obtained from the 3D image 400 of the oral cavity. The first library teeth 440 may be a 3D image including all library teeth corresponding to the 3D image of the oral cavity. For example, the first library teeth 440 may be the 3D image including all library teeth from tooth number 1 to tooth number 32.

In the present disclosure, second library teeth may include at least a portion of the first library teeth and, for ease of description, they are distinguished. The second library teeth may be library teeth corresponding to a plurality of selected teeth.

According to an embodiment, the electronic device 100 may generate the first library teeth. A tooth may include a crown region (e.g., region exposed from gingiva) and a root region (e.g., region hidden by gingiva). Based on mesh information of the crown region of a library tooth, the electronic device 100 may enlarge the library tooth in the direction of a gingival region. An actual tooth exists from a crown region to a root region and thus, the electronic device 100 may form a root region by extending to the direction of the gingival region so that the library tooth has a similar shape to the shape of the actual tooth. The 3D image of the oral cavity may not include the root region. In the case of the actual tooth, the root region is hidden by gingiva, and thus the root region may not be included in a scanning result. Therefore, the electronic device 100 may obtain the tooth region and the gingival region from the 3D image of the oral cavity, as a result of segmentation. In this instance, the tooth region in the 3D image of the oral cavity may be the crown region of a tooth. The electronic device 100 may generate the crown region of the library tooth based on the tooth region of the 3D image of the oral cavity. Based on the mesh information of a portion of the crown region, the first library teeth may be enlarged in the direction of the gingival region. Through the above, the electronic device 100 may also represent the root region when generating the first library teeth.

The electronic device 100 may enlarge the library tooth in the direction of the gingival region based on the mesh information of a region 461 that is at least a portion of the library tooth in the tooth region. For example, the electronic device 100 may elongate the mesh of the region 461 in the direction of the gingival region based on a central axis that vertically crosses the tooth, so as to form the root region. The mesh information of an enlarged region 462 may be the same as the mesh information of the region 461. The enlarged region 462 may be used to represent the root region of the library tooth. When the electronic device 100 enlarges the library tooth, so that the library tooth overlaps the 3D image 400, no empty space between the tooth region and the gingival region is visible. In this manner, the 3D image of the oral cavity overlapped with the library tooth may facilitate a realistic simulation, and may reduce confusion of a worker so that high work productivity is achieved, and the amount of computing resources consumed to obtain an equivalent result may be decreased.

According to another embodiment, the library tooth may represent the crown region and may not represent the root region. When the library tooth represents only the crown region, the height of a dental prosthesis included in the library tooth may be lower than an actual dental prosthesis to be placed. In this instance, a user may need to arbitrarily correct the height of the dental prosthesis. Therefore, for a realistic simulation, the electronic device may enlarge the library tooth based on mesh information of the crown region of the library tooth so as to represent the root region in addition to the crown region of the library tooth.

According to an embodiment, the electronic device 100 may process the enlarged region of the enlarged first library teeth based on a plurality of margin lines. For example, the electronic device 100 may change mesh information of a margin line 450 so that an existing region and an enlarged region are distinguished based on the corresponding margin line 450. The electronic device 100 may apply an implicit surfacing scheme to the crown region and the root region of the library tooth so as to smoothly represent a form in which the crown region and the root region are connected. The implicit surfacing is a scheme that defines a surface based on a point at which a function value of a mathematical function, particularly, a function value at an arbitrary point (x, y, z), is a predetermined value. This scheme defines a surface mathematically, and thus it may ensure continuity and smoothness of the surface. In addition, by using the implicit surfacing scheme, local details of a model may be efficiently processed.

For example, the electronic device 100 may remove a region in a direction of the gingival region from the library tooth based on the margin line of the library tooth so as to generate a tooth shape having an actually needed length. The electronic device 100 may process the library tooth to be similar to an actual tooth form.

In an embodiment, according to determination indicating that a plurality of teeth includes at least one of a removed tooth or a prep tooth, the electronic device 100 may generate library teeth including a dental prosthesis corresponding to the plurality of teeth. The removed tooth in dental treatment may be a tooth that is removed by external factors or via a dental procedure. The prep tooth may be a tooth that is prepared in advance for dental restoration or operation. The prep may include a form that is made by carving or sculpting a tooth variously so as to suit a predetermined requirement. The prep tooth is essential for placing various types of dental prostheses such as a crown, bridge, veneer, or the like. The dental prosthesis is a medical device designed for replacing a removed tooth or restoring a damaged tooth. The prosthesis may include a crown, bridge, dentures, or the like, and may be manufactured according to a predetermined purpose and design. The crown is for enclosing and protecting a damaged tooth, and the bridge is for filling a missing part using an abutment tooth. The dentures are used for dental functions and aesthetics for a patient with some or no teeth remaining. The electronic device 100 may recognize that a tooth corresponding to a predetermined tooth number is a removed tooth or a prep tooth via the 3D image of the oral cavity. As another example, the electronic device 100 may determine, based on a user input, that a plurality of teeth is at least one of a removed tooth or a prep tooth.

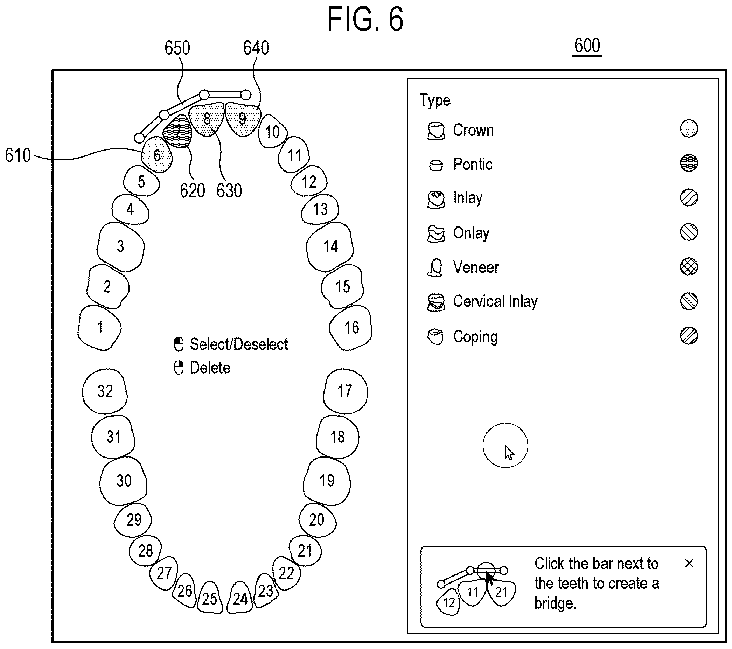

According to an embodiment, the electronic device 100 may receive an input for selecting a plurality of teeth of the target oral cavity. Based on the 3D image of FIGS. 4A and 4B, the user may determine a tooth for which a library tooth is to be generated and arranged. The input for selecting the plurality of teeth will be described with reference to FIG. 6.

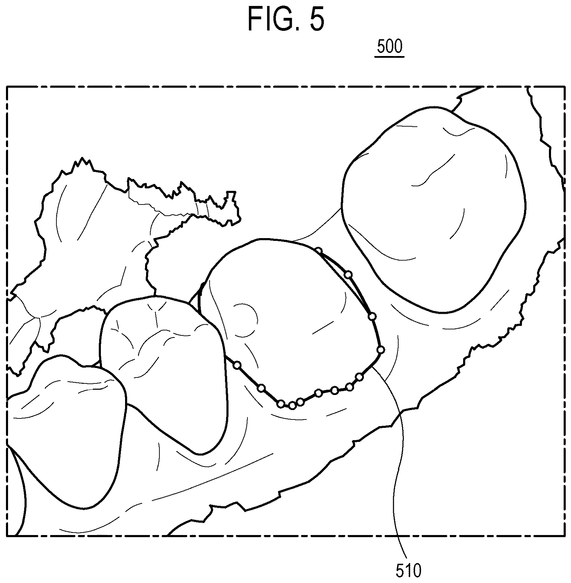

FIG. 5 is a diagram illustrating a margin line according to the present disclosure.

The margin line shows a boundary at which an intact tooth begins to emerge from a gingival region of a 3D image of a target oral cavity. For the intact tooth of the target oral cavity, the margin line may be a boundary line between the gingival region and a tooth region of the 3D image of the oral cavity. For example, as illustrated in FIG. 5, a margin line 510 may be displayed in the 3D image 400 of the oral cavity. On the other hand, in the case of a removed tooth or a prep tooth of the target oral cavity, a margin line may be generated by assuming that an intact tooth exists in the corresponding location.

According to an embodiment, the electronic device 100 may obtain a plurality of margin lines respectively corresponding to a plurality of teeth. The electronic device 100 may obtain the margin line based on a boundary between the tooth region and the gingival region obtained as a result of segmentation. According to another embodiment, the electronic device 100 may configure, based on a user input, margin lines in the 3D image of the oral cavity that respectively correspond to a plurality of teeth. Through the above, a user may edit the margin lines that the electronic device 100 automatically configures.

In an embodiment, the electronic device 100 may arrange the first library teeth 440 in a 3D space. The first library teeth 440 may be placed in the 3D space identical to the 3D image 400 of the oral cavity, but may be arranged to be spaced apart from the 3D image 400 of the oral cavity. The first library teeth 440 may be omitted or may be displayed on a screen.

In an embodiment, the electronic device 100 may arrange, based on the plurality of margin lines, the first library teeth 440 in the 3D space. The electronic device 100 may use the plurality of margin lines so that the first library teeth 440 are arranged to be the same as an oral structure of the 3D image 400 of the oral cavity in the 3D space. The plurality of margin lines may respectively correspond to tooth numbers. For example, in the 3D image of the oral cavity, there may be margin line A corresponding to tooth number 1, and margin line B corresponding to tooth number 2. The margin lines may include arrangement information of the plurality of teeth in the 3D image of the oral cavity, respectively. For example, the positions of the margin lines in the 3D image of the oral cavity may indicate positions of the teeth in a dental arch. Therefore, the electronic device 100 may arrange the first library teeth in the 3D space by matching the margin lines and the tooth numbers of the library teeth. For example, margin line A may correspond to tooth number 1 in the library teeth and margin line B may correspond to tooth number 2. Therefore, the electronic device 100 may store a margin line corresponding to each of the first library teeth as position information of a 3D tooth image. The position information may include the center point of the margin line. The electronic device 100 may arrange the first library teeth in the 3D space based on the position information (e.g., margin line) corresponding to each of the first library teeth.

In another embodiment, the electronic device 100 may generate a two dimensional (2D) image of the oral cavity based on the 3D image of the oral cavity. The electronic device 100 may generate the 2D image of the oral cavity corresponding to a tooth region by using the tooth region obtained from the 3D image of the oral cavity as a result of segmentation. The electronic device 100 may identify a plurality of teeth included in the 2D image by using a tooth number identification model, and may determine 2D center coordinates for each tooth. For example, the electronic device 100 may determine 2D center coordinates of tooth number 6, 2D center coordinates of tooth number 7, or the like. The tooth number identification model may be an artificial neural network model, and may perform an inference process that receives a 2D image of an oral cavity and identifies teeth from the 2D image of the oral cavity. The electronic device 100 may identify each of the plurality of teeth from the 2D image by using the tooth number identification model. The electronic device 100 may determine a tooth number for each of the plurality of teeth in the 2D image by using the tooth number identification model. The tooth number may be determined based on a system (e.g., Federation Dentaire International (FDI) or universal numbering system) for classifying a plurality of teeth. For example, the tooth number identification model may receive the 2D image, and may output a plurality of tooth numbers from the 2D image based on at least one of the shape of each tooth in the 2D image and a color indicating a curvature. The tooth number identification model may be a model that is trained on a correlation between a 2D image and tooth numbers. The electronic device 100 may identify the plurality of teeth from the 2D image based on the inference result obtained by the tooth number identification model including the tooth numbers of the teeth. The electronic device 100 may arrange the first library teeth in the 3D space by using position information corresponding to the tooth numbers in the 2D image.

In another embodiment, the electronic device 100 may arrange the first library teeth 440 in the 3D space by using the tooth region obtained as a result of segmentation. The electronic device 100 may identify a tooth number corresponding to a tooth region obtained as a result of segmentation. The electronic device 100 may compare a first tooth shape corresponding to the tooth region of the 3D image of the oral cavity and a second tooth shape corresponding to a tooth number by using the tooth number identification model, and may identify a tooth number having the most similar shape to the first tooth shape. The tooth number identification model may be a model trained to obtain similarity between images. The tooth number identification model may identify the second tooth shape similar to the first tooth shape which is the input 3D image, and may output a predetermined tooth number corresponding to the second tooth shape. The tooth number identification model may be a model that is trained on a correlation between a tooth shape that is a 3D image and a tooth number. In an embodiment, the electronic device 100 may arrange, based on the tooth numbers, the first library teeth 440 in the 3D space. The tooth numbers may indicate position information of the teeth in the dental arch. Therefore, the electronic device 100 may be aware of position information corresponding to a predetermined tooth number. For example, the electronic device 100 may be aware of a position where tooth number 6 is placed in the dental arch, and a position where tooth number 7 is placed in the dental arch. The electronic device 100 may arrange the first library teeth in the 3D space by using position information corresponding to the tooth numbers. Through the above, the first library teeth 440 may be arranged to be the same as the oral structure of the 3D image 400 of the oral cavity in the 3D image.

FIG. 6 is a diagram illustrating a method of selecting a plurality of teeth according to the present disclosure.

On a screen 600, a tooth arrangement and tooth numbers may be displayed. A user may select a plurality of tooth numbers. The electronic device 100 may receive an input for selecting a tooth number for each of a plurality of teeth of a target oral cavity. For example, the user may select tooth number 6 610, tooth number 7 620, tooth number 8 630, and tooth number 9 640 that are adjacent to each other. As another example, the user may also select tooth numbers 1, 5, 14, and 19 that are not adjacent to each other.

In an embodiment, the electronic device 100 may receive an input for selecting a plurality of teeth, and may display library teeth corresponding to the selected tooth numbers so as to overlap the 3D image 400 of the oral cavity.

Via the screen 600, the user may select a type of dental prosthesis to be generated for a selected tooth number. For example, when the user selects a tooth number and selects a crown, post processing may be performed so that a corresponding library tooth is arranged on the corresponding tooth and data for manufacturing a crown is generated. As another example, when the user selects a tooth number and selects an inlay, post processing may be performed so that a corresponding library tooth is arranged on the corresponding tooth and data for manufacturing an inlay is generated. In addition, the user may add a connector 650 for the selected tooth number.

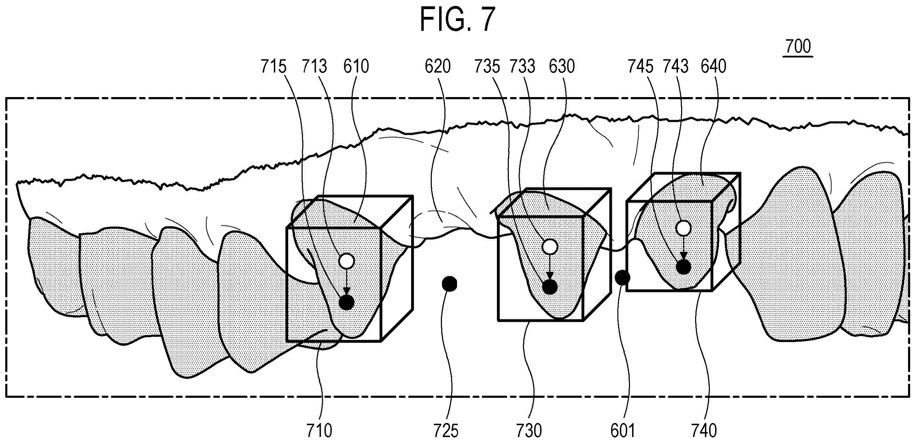

FIG. 7 is a diagram illustrating second position information in a 3D image of an oral cavity according to the present disclosure.

Second library teeth and the 3D image may be arranged in different planes. Therefore, the electronic device 100 may move the second library teeth corresponding to a plurality of teeth so that the second library teeth are arranged in a space corresponding to the plurality of teeth in the 3D image of the oral cavity. To this end, the electronic device 100 may use first position information corresponding to each of the second library teeth and second position information of each tooth of the 3D image of the oral cavity.

In an embodiment, the electronic device 100 may move a plurality of second library teeth corresponding to the plurality of teeth among first library teeth in a 3D space so that the plurality of second library teeth is arranged in a space corresponding to the plurality of teeth in the 3D image of the oral cavity.

In an embodiment, the electronic device 100 may generate second position information including the center point of a second bounding box for each tooth of the 3D image of the oral cavity. The second position information may be generated for each tooth included in the 3D image of the oral cavity. For example, tooth number 6 610, tooth number 8 630, and tooth number 9 640 may correspond to second bounding box A 710, second bounding box C 730, and second bounding box D 740, respectively. The bounding box refers to the minimum enclosing rectangular outline including an object, and is mainly used for computer graphic and data analysis. A 3D bounding box is an extension of this concept to a 3D space. This may be defined as the narrowest 3D rectangular parallelepiped including a specified object. A first bounding box is associated with a library tooth, and a second bounding box is associated with a tooth included in the 3D image of the oral cavity.

In another embodiment, the electronic device 110 may enable second position information of a removed tooth to include a point in a dental arch that corresponds to a tooth number. For the removed tooth, a second bounding box may not be generated. Therefore, the electronic device 110 may determine the point where the tooth number corresponding to the removed tooth is placed in the dental arch as the second position information of the removed tooth. For example, when tooth number 7 620 is a removed tooth, the electronic device 100 may determine to include, in second position information, a point 725 corresponding to tooth number 7 in the dental arch. By placing the point corresponding to the removed tooth in the dental arch, the electronic device 100 may represent a library tooth corresponding to the removed tooth so as to overlap a normal position in the 3D image of the oral cavity.

The dental arch may be represented as a curved line. The electronic device 100 may determine the dental arch based on the center point of the second bounding box of each tooth of the 3D image of the oral cavity. The position and the shape of the dental arch may be determined to pass through the largest number of center points of the second bounding boxes. The curved line that represents the dental arch may not be displayed on a screen.

In another embodiment, the electronic device 110 may store second position information by changing the center point of a second bounding box of a prep tooth. Based on a difference between the height of the highest point of the prep tooth and the height of the highest point of a library tooth corresponding to the prep tooth, the electronic device 100 may store the second position information in which the center point of the second bounding box is changed. For example, the teeth respectively corresponding to tooth number 6 610, tooth number 8 630, and tooth number 9 640 may be prep teeth. The center points of second bounding box A 710, second bounding box C 730, and second bounding box D 740 may be center points 713, 733, and 743, respectively. However, in terms of the characteristics of a prep tooth, a prep tooth may have a higher or lower height than a normal tooth. Therefore, when the first position information of the library tooth is changed based on the center point of the second bounding box of the prep tooth, a large portion of the library tooth may be hidden by a gingival region. Therefore, the electronic device 100 may change the second position information of the prep tooth to include a corrected point as opposed to the center point of the second bounding box. For example, the electronic device 100 may move the center point of the second bounding box by the difference between the height of the highest point of the prep tooth and the height of the highest point of the library tooth corresponding to the prep tooth. For example, the electronic device 100 may move the center points 713, 733, and 743 that are center points of second bounding box A 710, second bounding box C 730, and second bounding box D 740 to positions 715, 735, and 745, respectively, and may include the corresponding points in the second position information. For example, the difference in height between the points 713 and 715 may be the difference between the height of the highest point of tooth number 6 610 and the height of the highest point of library tooth number 6.

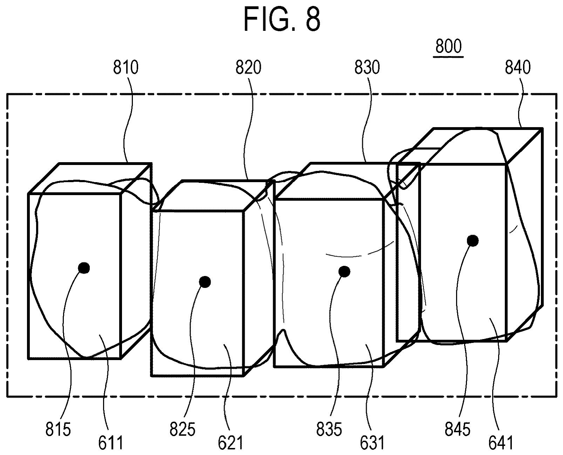

FIG. 8 is a diagram illustrating first position information of a library tooth according to the present disclosure.

In an embodiment, based on each of a plurality of margin lines, the electronic device 100 may generate first position information including the center point of a first bounding box for each of a plurality of library teeth. The first position information may refer to position information of a library tooth in a 3D space. First library teeth may be arranged based on the plurality of margin lines. Therefore, the plurality of library teeth may be arranged to be the same as a dental structure in a 3D image of an oral cavity in the 3D space. Upon placement, each library tooth may have coordinates in the 3D space. As an example, a bounding box may be used to determine the coordinates of the library tooth. The electronic device 100 may generate a first bounding box for each of the plurality of library teeth (e.g., second library teeth). For example, the electronic device 100 may generate first bounding box A 810, first bounding box B 820, first bounding box C 830, and first bounding box D 840, respectively, for tooth number 6 610, tooth number 7 620, tooth number 8 630, and tooth number 9 640. The electronic device 100 may include the center point of a first bounding box in first position information. For example, the electronic device 100 may include, in first position information, a center point 815 of the first bounding box A 810 of library tooth number 6 611 corresponding to tooth number 6 610. In the same manner, the first position information of library tooth number 7 621 may include a center point 825 of first bounding box B 820, the first position information of library tooth number 8 631 may include a center point 835 of first bounding box C 830, and the first position information of a library tooth number 9 641 may include a center point 845 of first bounding box D 840.

In an embodiment, a library tooth corresponding to a removed tooth may be a basis for the shape of a dental prosthesis corresponding to the removed tooth. For example, referring to FIG. 7, tooth number 7 620 may be a removed tooth. Therefore, library tooth number 7 621 may be a basis for the shape of the removed tooth.

In an embodiment, a library tooth corresponding to a prep tooth may be a basis for the shape of a dental prosthesis corresponding to the prep tooth. For example, referring to FIG. 7, tooth number 6 610, tooth number 8 630, and tooth number 9 640 may be prep teeth. Therefore, library tooth number 6 611, library tooth number 8 631, and library tooth number 9 641 may be bases for the shapes of prostheses corresponding to the prep teeth.

In an embodiment, according to determination indicating that a plurality of library teeth corresponding to a plurality of teeth is connected via a connector, the electronic device 100 may include a connector between the library teeth. For example, according to determination indicating that library tooth number 6 611, library tooth number 7 621, library tooth number 8 631, and library tooth number 9 641 are connected via a connector, the electronic device 100 may represent the connector between the library teeth as a 3D image. The connector may be represented based on mesh information. A bridge is one of dental treatments used for replacing a removed tooth. Unlike a prep tooth, in the case of a removed tooth, an object to which a dental prosthesis is to be attached does not exist, and thus the bridge is provided in a structure that retains an artificial tooth by using an abutment tooth as support. The bridge may include a connector that connects dental prostheses. The bridge may be made of metal, porcelain, or a composite material thereof, and may be provided in various forms depending on the need and oral cavity state of a patient.

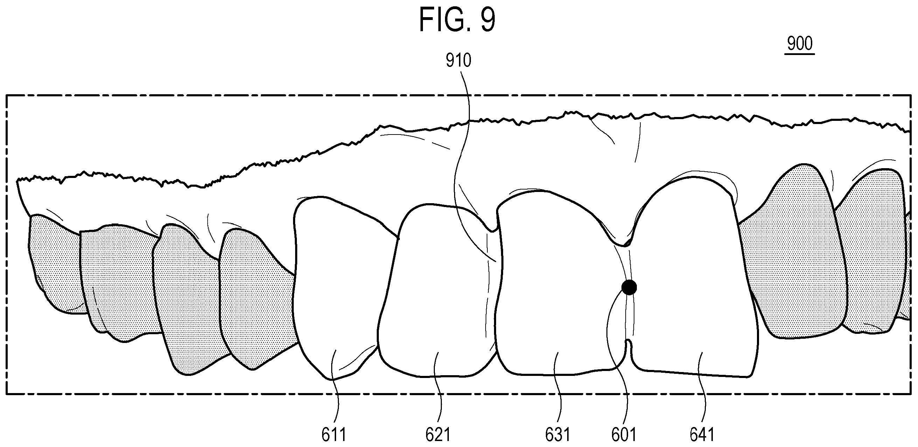

FIG. 9 is a diagram illustrating a method of overlapping the library tooth described with reference to FIG. 8 to the 3D image of the oral cavity described with reference to FIG. 7.

In an embodiment, the electronic device 100 may move, based on the origin (e.g., the origin in the dental arch of the oral cavity), a library tooth (e.g., second library tooth) to a position of a tooth with the same tooth number in the 3D image of the oral cavity.

In an embodiment, the electronic device 100 may generate, based on the origin in the dental arch of the oral cavity, a correlation between first position information and second position information of each of a plurality of second library teeth. The correlation between the first position information and the second position information may be generated for each tooth. For example, the correlation between the first position information and second position information for each of tooth numbers 1, 2, ..., 32 may be generated. The dental arch of the oral cavity refers to an anatomical structure related to a tooth arrangement in which maxillary and mandibular teeth are arranged, and may include a form in which the teeth are consecutively arranged in an arch form. The maxilla and mandible are distinguished as the maxillary arch and mandibular arch, respectively, and each dental arch is symmetrical with respect to a midline. The origin in the arch may be determined based on a point between two central incisors. For example, a point between tooth number 8 and tooth number 9 may be determined as the origin in the maxillary arch, and a point between tooth number 24 and tooth number 25 may be determined as the origin in the mandibular arch. As another example, the origin may be an intersecting point between the arch and the midline. The origin may be different for each of the maxilla and the mandible. For example, when a tooth exists in the maxilla, the electronic device 1000 may generate a correlation based on the origin of the maxilla. When a tooth exists in the mandible, the electronic device 100 may generate a correlation based on the origin of the mandible.

In an embodiment, the electronic device 100 may generate, based on the origin, a tooth-based transform matrix indicating a correlation between first position information of each of a plurality of library teeth and second position information having the same tooth number as each of the plurality of second library teeth. The tooth-based transform matrix may be a matrix that moves a point included in the first position information to the origin, and moves the corresponding point to a point included in the second position information.

For example, the transform matrix of tooth number 6 610 may indicate, based on an origin 601 of the maxillary arch, a correlation between the first position information of the library tooth number 6 611 and the second position information of tooth number 6 610 in the 3D image of the oral cavity. As another example, the transform matrix of tooth number 8 630 may indicate, based on the origin of the maxillary arch, a correlation between the first position information of library tooth number 8 631 and the second position information of tooth number 8 630 in the 3D image of the oral cavity.

In an embodiment, the electronic device 100 may display the 3D image of the target oral cavity and the plurality of second library teeth to overlap each other. The electronic device 100 may display the plurality of second library teeth so as to overlap, based on the tooth-based transform matrix, the 3D image of the oral cavity with teeth respectively having the same tooth numbers as the plurality of second library teeth in a 3D space. Specifically, by applying the tooth-based transform matrix to all points of a mesh included in a second library tooth, the all points of the mesh included in the second library tooth may be arranged in the 3D image of the oral cavity. For example, the electronic device 100 may apply the transform matrix of tooth number 6 to all points of a mesh included in library tooth number 6 611, so as to move the all points of the mesh included in library tooth number 6 611 to a place where tooth number 6 is placed in the 3D image of the oral cavity.

Through the above, the electronic device 100 may display the library tooth number 6 611 so as to overlap the position (e.g., position based on second position information) of tooth number 6 610 in the 3D image of the oral cavity. In the same manner, the electronic device 100 may display library tooth numbers 7, 8, and 9 621, 631, and 641, respectively, so as to overlap the positions of tooth numbers 7, 8, and 9 620, 630, and 640, respectively, in the 3D image of the oral cavity, respectively.

In the present disclosure, the operation of displaying a plurality of library teeth overlappingly in the 3D image of the oral cavity is overlappingly displaying library teeth in the state of maintaining the 3D image of the oral cavity, and when the library teeth are moved, the existing teeth in the 3D image of the oral cavity may be displayed.

In an embodiment, the electronic device 100 may display, based on a tooth-based transform matrix corresponding to a removed tooth, a library tooth corresponding to the removed tooth so as to overlap the 3D image of the target oral cavity. For example, when the removed tooth is tooth number 7 620, the electronic device 100 may move the library tooth number 7 621 to a position of tooth number 7 620 based on the transform matrix of tooth number 7 620.

In an embodiment, the electronic device 100 may display, based on a tooth-based transform matrix corresponding to a prep tooth, a library tooth corresponding to the prep tooth so as to overlap the 3D image of the target oral cavity. For example, when the prep tooth is tooth number 6 610, the electronic device 100 may move the library tooth number 6 611 to a position of tooth number 6 610 based on the transform matrix of tooth number 6 610.

In an embodiment, according to determination indicating that a plurality of library teeth corresponding to a plurality of adjacent teeth is connected via a connector, the electronic device 100 may display the connector between the plurality of library teeth. For example, according to determination indicating that library tooth number 7 621 and library tooth number 8 631 are connected via a connector, the electronic device 100 may display a connector 910 so as to connect library tooth number 7 621 and library tooth number 8 631.

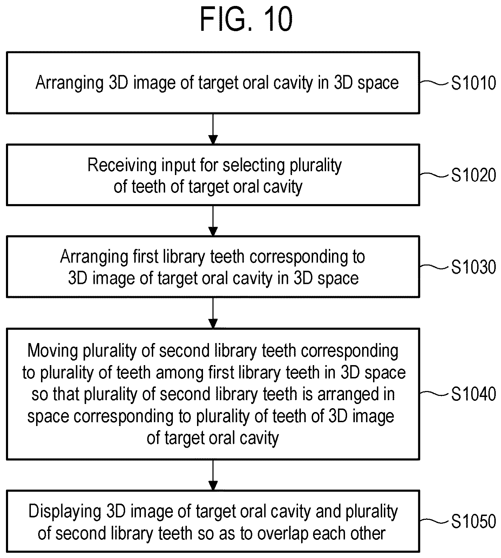

FIG. 10 is a flowchart illustrating a method of displaying a library tooth so as to overlap a 3D image of an oral cavity, by a server according to the present disclosure.

In an embodiment, the server may arrange a 3D image of a target oral cavity in a 3D space in operation S1010.

In an embodiment, the server may receive an input for selecting a plurality of teeth of the target oral cavity in operation S1020.

In an embodiment, the server may arrange first library teeth corresponding to the 3D image of the target oral cavity in the 3D space in operation S1030.

In an embodiment, the server may move a plurality of second library teeth corresponding to the plurality of teeth among the first library teeth in the 3D space so that the plurality of second library teeth is arranged in a space corresponding to the plurality of teeth in the 3D image of the oral cavity in operation S1040.

In an embodiment, the server may display the 3D image of the target oral cavity so as to overlap the plurality of second library teeth in operation S1050.

Various embodiments of the present disclosure and their effects have been described with reference to FIGS. 1 to 10. The effects of the technical spirit of the present disclosure are not limited to the above-mentioned effects and other effects which have not been mentioned above will be clearly understood by those skilled in the art from the description.

The technical spirit that has been described with reference to FIGS. 1 to 10 may be implemented by computer readable codes in a recording medium readable by a computing device (e.g., electronic device). Here, the computer program implemented by codes, when loaded into a memory of the computing device, may include at least one instruction that causes a processor of the computing device to perform an operation according to various embodiments of the present disclosure. The computer-readable recording medium may be, for example, a removable recording medium (e.g., CD, DVD, Blu-ray disc, USB storage device, a removable hard disk, etc.) or a fixed recording medium (e.g., ROM, RAM, hard disk drive included in computer, etc.). In addition, the recording medium may be a non-transitory recording medium. Here, the non-transitory recording medium may be a tangible medium irrespective of whether data is semi-permanently or temporarily stored, and may exclude a signal propagated in a transitory manner. Furthermore, the computer program stored in the recording medium may be transmitted to another computing device via a network such as the Internet or the like and may be installed in the other computing device, thereby being used in the other computing device.

Although the technical spirit of the present disclosure has been described by the examples described in some embodiments and illustrated in the accompanying drawings, it should be noted that various substitutions, modifications, and changes can be made without departing from the scope of the present disclosure which can be understood by those skilled in the art to which the present disclosure pertains. In addition, it should be noted that such substitutions, modifications and changes are intended to fall within the scope of the appended claims.

## Claims

1. An image processing method performed by an electronic device, the method comprising:
arranging a three-dimensional (3D) image of a target oral cavity in a 3D space;
receiving an input for selecting a plurality of teeth of the target oral cavity;
arranging first library teeth corresponding to the 3D image of the target oral cavity in the 3D space;
moving a plurality of second library teeth in the 3D space such that the plurality of second library teeth corresponding to the plurality of teeth among the first library teeth are arranged in a space corresponding to the plurality of teeth among the 3D image of the target oral cavity; and
displaying the 3D image of the target oral cavity and the plurality of second library teeth to overlap each other.

2. The method of Claim 1, further comprising obtaining a plurality of margin lines corresponding to the plurality of teeth respectively,
wherein the arranging the first library teeth in the 3D space comprises:
based on the plurality of margin lines, arranging the first library teeth in the 3D space, and
wherein each of the plurality of margin lines is a boundary at which an intact tooth begins to emerge from a gingival region of the 3D image of the target oral cavity.

3. The method of Claim 2, wherein the obtaining the plurality of margin lines comprises:
based on a boundary between a tooth region and a gingival region obtained as a result of segmentation, obtaining the plurality of margin lines; or
based on a user input, obtaining the plurality of margin lines.

4. The method of Claim 1, wherein the arranging the first library teeth in the 3D space comprises:
identifying a tooth number corresponding to a tooth region obtained as a result of segmentation; and
based on the tooth number, arranging the first library teeth in the 3D space.

5. The method of Claim 1, further comprising generating the first library teeth.

6. The method of Claim 5, wherein the generating the first library teeth comprises:
based on mesh information of at least a portion of the first library teeth corresponding to a tooth region among the tooth region and a gingival region included in the 3D image of the target oral cavity, enlarging the first library teeth in a direction of the gingival region.

7. The method of Claim 6, wherein the generating the first library teeth further comprises:
based on the plurality of margin lines, processing the enlarged region in the enlarged first library teeth.

8. The method of Claim 1, wherein the receiving the input for selecting the plurality of teeth of the target oral cavity comprises:
receiving an input for selecting a tooth number for each of the plurality of teeth of the target oral cavity.

9. The method of Claim 1, wherein the moving the plurality of second library teeth in the 3D space comprises:
based on each of the plurality of margin lines, generating first position information including a center point of a first bounding box for each tooth of the plurality of second library teeth;
generating second position information including a center point of a second bounding box for each tooth of the 3D image of the target oral cavity; and
based on an origin positioned in a dental arch of the target oral cavity, generating a correlation between the first position information and the second position information for each of the plurality of second library teeth.

10. The method of Claim 9, wherein the generating the correlation comprises:
based on the origin, generating a tooth-specific transform matrix indicating a correlation between the first position information of each of the plurality of second library teeth and the second position information having the same tooth numbers as each of the plurality of the second library teeth.

11. The method of Claim 10, wherein the displaying the 3D image of the target oral cavity and the plurality of second library teeth to overlap each other comprises,
based on the tooth-specific transform matrix, displaying the plurality of second library teeth to overlap the 3D image of the target oral cavity having the same tooth numbers as the plurality of second library teeth, respectively, in the 3D space.

12. The method of Claim 11, wherein the origin is a point between two central incisors in the dental arch.

13. The method of Claim 1, wherein the displaying the 3D image of the target oral cavity and the plurality of second library teeth to overlap each other comprises,
based on a tooth-specific transform matrix corresponding to a removed tooth, displaying a library tooth corresponding to the removed tooth to overlap the 3D image of the target oral cavity.

14. The method of Claim 13, wherein the tooth-specific transform matrix corresponding to the removed tooth is generated based on second position information including a point in a dental arch that corresponds to the tooth number, and
wherein the library tooth corresponding to the removed tooth comprises a dental prosthesis corresponding to the removed tooth.

15. The method of Claim 1, wherein the displaying the 3D image of the target oral cavity and the plurality of second library teeth to overlap each other comprises,
based on a tooth-specific transform matrix corresponding to a prep tooth, displaying a library tooth corresponding to the prep tooth to overlap the 3D image of the target oral cavity.

16. The method of Claim 15, wherein the tooth-specific transform matrix corresponding to the prep tooth is generated based on second position information of which a center point of a second bounding box is changed based on a difference between a height of a highest point of the prep tooth and a height of a highest point of the library tooth corresponding to the prep tooth, and
wherein the library tooth corresponding to the prep tooth comprises a dental prosthesis corresponding to the prep tooth.

17. The method of Claim 1, wherein the displaying the 3D image of the target oral cavity and the plurality of second library teeth to overlap each other comprises,
based on a determination that the plurality of second library teeth corresponding to the plurality of teeth is connected via a connector, displaying the connector between the plurality of second library teeth.

18. An electronic device comprising:
a processor;
a network interface communicatively connected to an intraoral scanner;
a display;
a memory; and
a computer program loaded into the memory and executed by the processor,
wherein the computer program is configured to:
arrange a three-dimensional (3D) image of a target oral cavity in a 3D space;
receive an input for selecting a plurality of teeth of the target oral cavity;
arrange first library teeth corresponding to the 3D image of the target oral cavity in the 3D space;
move a plurality of second library teeth in the 3D space such that the plurality of second library teeth corresponding to the plurality of teeth among the first library teeth are arranged in a space corresponding to the plurality of teeth among the 3D image of the target oral cavity; and
display the 3D image of the target oral cavity and the plurality of second library teeth to overlap each other.

19. A non-transitory computer-readable recording medium recording a computer program to be executed by a processor, the computer program comprising:
an instruction for arranging a three-dimensional (3D) image of a target oral cavity in a 3D space;
an instruction for receiving an input for selecting a plurality of teeth of the target oral cavity;
an instruction for arranging first library teeth corresponding to the 3D image of the target oral cavity in the 3D space;
an instruction for moving a plurality of second library teeth in the 3D space such that the plurality of second library teeth corresponding to the plurality of teeth among the first library teeth are arranged in a space corresponding to the plurality of teeth of the 3D image of the target oral cavity; and
an instruction for displaying the 3D image of the target oral cavity and the plurality of second library teeth to overlap each other.
